# EUROPÄISCHE PATENTSCHRIFT

(11) **EP 2 482 858 B1**
(45) Veröffentlichungstag und Bekanntmachung des Hinweises auf die Patenterteilung: **02.04.2014**
(21) Anmeldenummer: 10737506.5
(22) Anmeldetag: 20.07.2010
(51) Int. Cl.: A61L 2/10, B65B 55/08

(54) **VERFAHREN UND VORRICHTUNG ZUR STERILISATION VON OBERFLÄCHEN**
METHOD AND APPARATUS FOR STERILIZING SURFACES
PROCÉDÉ ET DISPOSITIF DE STÉRILISATION DE SURFACES

(30) Priorität: 30.09.2009 DE 102009043496; 01.10.2009 DE 102009043726
(43) Veröffentlichungstag der Anmeldung: 08.08.2012
(73) Patentinhaber: KHS GmbH, 44143 Dortmund (DE)
(72) Erfinder: KEIL, Gernot, 55595 Braunweiler (DE)
(86) Internationale Anmeldenummer: PCT/EP2010/004411
(87) Internationale Veröffentlichungsnummer: WO 2011/038799

(56) Entgegenhaltungen:
- EP-A2- 0 341 069
- WO-A1-97/29016
- DE-A1- 2 530 099
- DE-A1-102006 026 278
- US-A- 4 910 942

## Beschreibung

Die Erfindung betrifft ein Verfahren zur Sterilisation von Oberflächen, insbesondere von Verpackungsgutoberflächen, beispielsweise Oberflächen von Verpackungsbahnen, Hohlkörpern oder dergleichen, nach dem Oberbegriff von Anspruch 1.

Kurzwellige elektromagnetische Strahlung meint im Rahmen der Erfindung Strahlung, die sich regelmäßig im kurzwelligen Bereich an das sichtbare Spektrum anschließt, also im Allgemeinen mit Wellenlängen unterhalb von 400 nm ausgerüstet ist. Bei der fraglichen kurzwelligen elektromagnetischen Strahlung handelt es sich im Regelfall um UV-Strahlung im Wellen-Längenbereich zwischen ca. 200 nm bis 380 nm. Die sterillsierende Wirkung solcher kurzwelliger elektromagnetischer Strahlung ist gut bekannt, weshalb UV-Strahlung zur desinfizierenden Behandlung von Wasser, Luft und Oberflächen eingesetzt wird (vgl. DE 10 2004 032 881 A1).

Dabei wird allgemein eine hohe Reaktionsgeschwindigkeit zwischen abzutötenden Mikroben und der desinfizierenden UV-Strahlung beobachtet, meistens in Bruchteilen einer Sekunde. D. h., die fraglichen Mikroben werden in dieser kurzen Zeit desinfiziert und der gewünschte Gegenstand sterilisiert. Dadurch können UV-Strahler beispielsweise auch zur Desinfektion von Luftströme eingesetzt werden. Außerdem kennt man die Trinkwasseraufbereitung mit UV-Strahlung. Hierbei wird die Keimzahl im Wasser zuverlässig und in Abhängigkeit von der Dosis stark reduziert, so dass eine Zugabe von Chemikalien grundsätzlich nicht erforderlich ist.

Die Sterillsation bzw. Desinfektion von Oberflächen und hier insbesondere von Verpeckungsgutoberflächen wird beispielsweise in der DE 39 02 432 A1 beschrieben. Hier geht es um ein Verfahren zum Reinigen und Sterilisieren von Verpackungsbehältern wie Flaschen, Ampullen oder dergleichen mittels eines in den Innenraum eingeblasenen Gases. Dazu erfolgt eine Bedampfung der Innenwandung der Behälter mit einem gegen den Boden gerichteten Dampfstrahl. Ganz abgesehen davon beschäftigt sich die Lehre nach der DE 195 20 925 A1 mit einem Verfahren zum keimarmen Füllen von Kunststoff-Flaschen, beispielsweise von PET-Flaschen. Hier wird jeder Behälter unmittelbar vor dem Verschließen an seiner Behältermündung mit einem Sterilisationsmedium, vorzugsweise mit Heißdampf, behandelt. Ergänzend erfolgt eine Beaufschlagung mit UV-C-Strahlen zur Keimtötung, Das hat sich grundsätzlich bewährt.

Ganz unabhängig davon ist es durch die DE 10 2004 029 803 B4 bekannt, zur Sterilisation von aus Kunststoff, Metall oder Glas bestehenden Flaschen, Dosen oder Behältern in einer Gefäßbehandlungsmaschine Wasserstoffperoxid (H₂O₂) einzusetzen. Dabei ist im Rahmen der bekannten Lehre im Strömungsweg mindestens ein die Zersetzung des H₂O₂ fördernder Katalysator angeordnet.

Die gattungsbildende Lehre nach der EP 0 341 069 A2 beschäftigt sich mit der Sterilisation von Oberflächen, wobei neben elektromagnetischer Strahlung zusätzlich Luft und Wasser als Transmissionsmedium zum Einsatz kommen. Außerdem wird in verschiedenen Beispielen Stickstoff zugesetzt.

Wie bereits zuvor erläutert, hängt die keimtötende Wirkung von kurzwelliger elektromagnetischer Strahlung und insbesondere von UV-Strahlung mehr oder minder stark von der Strahlungsdosis ab. Diese beschreibt die Intensität der Strahlung pro Zeit- und Flächeneinheit. In diesem Zusammenhang ist zu berücksichtigen, dass UV-Strahlung unterhalb von 200 nm so kurzweilig bzw. energiereich ist, dass sie durch molekularen Sauerstoff vollständig absorbiert wird. Dabei wird der molekulare Sauerstoff (O₂) in zwei freie Sauerstoffradikale (20) gespalten, die jeweils mit einem weiteren Molekül Sauerstoff (O₂) zu Ozon (O₃) weiter reagieren. Aus diesem Grund kann sich UV-Strahlung mit einer Wellenlänge unterhalb von 200 nm nur noch im Vakuum ausbreiten (sog. Volumen-UV-Strahlung).

Jedenfalls werden bei Anwendung eines UV-Strahlers oder einer UV-Strahlungsquelle in beispielsweise atmosphärischer Luft mehr oder minder ausgeprägte Absorptionen der emittierten UV-Strahlung oder allgemeln der kurzwelligen elektromagnetischen Strahlung beobachtet. Das kann unter Umständen zu einer reduzierten keimtötenden Wirkung an der zu behandelnden Oberfläche führen. Hier will die Erfindung insgesamt Abhilfe schaffen.

Der Erfindung liegt das technische Problem zugrunde, ein derartiges Verfahren zur Sterilisation von Oberflächen so weiter zu entwickeln, dass die keimabtötende bzw. sterilisierende Wirkung der auf die Oberfläche auftreffenden kurzwelligen elektromagnetischen Strahlung gegenüber bisherigen Vorgehensweisen verbessert ist. Zur Lösung dieser technischen Problemstellung schlägt die Erfindung bei einem gattungsgemäßen Verfahren zur Sterillsation von Oberflächen, insbesondere von Verpackungsgutoberflächen, vor, dass Wasser und/oder Wasserdampf den Transmissionsmedium und/oder der zu desinfizierenden Oberfläche zugeführt wird und das reine Gas oder die zweite Komponente ein Edelgas ist, welches in gasförmiger und/oder flüssiger Form der Luft und/oder der zu des infizierenden Oberfläche zugeführt wird. Im Rahmen der Erfindung kommt also ein spezielles Transmissionsmedium zwischen der Strahlungsquelle und der Oberfläche zum Einsatz. Tatsächlich zeichnet sich dieses Transmissionsmedium dadurch aus, dass es zu einer verringerten Absorption der hindurchgeführten elektromagnetischen Strahlung im Vergleich zu beispielsweise atmosphärischer Luft unter Normaldruck an dieser Stelle korrespondiert. Das Transmissionsmedium begünstigt also den ungehinderten Durchtritt der kurzwelligen elektromagnetischen Strahlung bzw. verringert die Absorption der Strahlung auf ihrem Weg von der Strahlungsquelle bis zur zu behandelnden Oberflächen respektive Verpackungsgutoberfläche. Anders ausgedrückt, wird der Durchtritt der elektromagnetischen Strahlung, die Transmission, gegenüber der Situation in atmosphärischer Luft verbessert.

Um dies im Detail zu erreichen, schlägt die Erfindung als Transmissionsmedium ein Flüssigkeits-/Gasgemisch vor. Das Flüssigkeits-/Gasgemisch enthält zu überwiegendem Tell keine atmosphärische Luft mehr. Denn zu beispielsweise der atmosphärischen Luft (unter Normaldruck) als erster Komponente tritt eine weitere zweite Komponente hinzu, die mehr als 50 Vol.-% eines von der Strahlung passierten Volumens, meistens sogar mehr als 80 Vol.-%, ausfüllt. Diese weitere zweite Komponente verfügt über eine geringere Absorption der kurzwelligen elektromagnetischen Strahlung im Vergleich zu der atmosphärischen Luft (unter Normaldruck), dadurch ist auch das Flüssigkeits-/Gasgemisch mit Luft als der ersten Komponente und der beschriebenen zweiten Komponente mit geringerer Absorption insgesamt so ausgelegt, dass die Transmission der elektromagnetischen Strahlung von der Strahlungsquelle bis zur Oberfläche begünstigt wird.

Dabei kann die besagte zweite Komponente in gasförmiger und/oder flüssiger Form beispielsweise der Luft und/oder der zu behandelnden Oberfläche direkt zugeführt werden. Für den Fall, dass die zweite Komponente in flüssiger.

Form vorliegt, empfiehlt es sich, die zu behandelnde Oberfläche zu beheizen. Denn auf diese Weise kann die Verdampfungsgeschwindigkeit der zweiten Komponente erhöht werden. Dabei reichen meistens oftmals geringe Mengen der zweiten Komponente in flüssiger Form aus. Beispielsweise empfiehlt die Erfindung als zweite Komponente Argon oder andere vergleichbare Edelgase wie Helium, Xenon, Krypton oder aber auch Stickstoff. Wenn man berücksichtigt, dass das Volumenverhältnis der vorerwähnten Gase von flüssigem zu festem Zustand mehrere 100 beträgt, beispielsweise für Argon 700 beträgt, so benötigt man für die vollständige Füllung beispielsweise einer Literflasche mit Argon lediglich 1,4 ml flüssigen Argon.

In jedem Fall verdampft die als zweite Komponente eingesetzte Flüssigkeit meistens kurzfristig in einem Zeitraum zwischen 1 und 5 Sek.. Bei diesem Vorgang mag das im Hohlkörper bzw. der Literflasche im Beispielsfall zunächst vorhandene Luftvolumen überwiegend verdrängt werden. Im Rahmen dieses Vorganges wird die Verdampfungsgeschwindigkeit dann noch erhöht, wenn die zu desinfizierende Oberfläche beheizt ist. - Die vorerwähnten Gase bzw. Schutzgase können auch unmittelbar und einzig als Transmissionsmedium in (zunächst) flüssiger und (dann) gasförmiger Form eingesetzt werden. Dann handelt es sich bei dem Transmissionsmedium nicht vorrangig um ein Gasgemisch, sondern lediglich das Gas bzw. Schutzgas.

Jedenfalls nutzt die Erfindung aus, dass die zuvor beschriebene zweite Komponente bzw. die an dieser Stelle eingesetzten Gase wie Argon, Helium, Xenon und Krypton als Edelgase oder auch Stickstoff allgemein als Schutzgase fungieren und die Ausbreitung der kurzwelligen elektromagnetischen Strahlung in dem Transmissionsmedium begünstigen. D. h., das Transmissionsmedium setzt sich im wesentlichen aus Luft als der ersten Komponente und einem überwiegenden Schutzgas in flüssiger oder gasförmiger Form als zweiter Komponente zusammen. Grundsätzlich kann es sich bei dem Transmissionsmedium aber auch nur um das Gas bzw. Schutzgas handeln.

Hierbei macht sich die Erfindung im Kern die bereits zuvor beschriebene Tatsache zunutze, dass die Ausbreitung elektromagnetischer Strahlung bei Wellenlängen beispielsweise unterhalb von 220 nm in Luft unter Normaldruck durch Absorption in oder an Sauerstoffmolekülen begrenzt ist. Denn die Sauerstoffmoleküle erfahren die zuvor beschriebene Aufspaltung in atomaren Sauerstoff mit anschließender Ozonbildung. Dieser Effekt ist im Rahmen der Erfindung zum Teil gewünscht, weil er die keimtötende Wirkung der kurzwelligen elektromagnetischen Strahlung durch die Bildung von Ozon unterstützt. Zugleich sorgt die zweite Komponente in dem Gasgemisch bzw. Flüssigkeits-/Gasgemisch bzw. das Schutzgas zur Darstellung des Transmissionsmediums jedoch dafür, dass die kurzwellige elektromagnetische Strahlung nicht (mehr) so stark wie in reiner atmosphärischer Luft absorbiert wird. Das ermöglicht das Schutzgas, welches von der fraglichen Strahlung mit deutlich höherer Intensität durchdrungen wird als ein vergleichbares Luftvolumen (bei Normaldruck). Auf diese Weise wird die Intensität der elektromagnetischen Strahlung an der zu sterilisierenden Oberfläche erhöht und folglich die sterilisierende Wirkung wie gewünscht verbessert.

Beispielsweise gilt für Argon, dass bis zu Wellenlängen von ca. 170 nm praktisch gar keine Transmissionsverluste der kurzwelligen elektromagnetischen Strahlung beobachtet werden. Folgerichtig kann Argon als Schutzgas bzw. zweite Komponente im Gasgemisch bzw. Flüssigkeits-/Gasgemisch neben der zwangsläufig vorhandenen atmosphärischen Luft als erster Komponente als optimal angesehen werden.

Darüber hinaus hat es sich als günstig erwiesen, wenn das Transmissionsmedium neben der atmosphärischen Luft und dem Schutzgas in gasförmiger oder flüssiger Form zusätzlich noch Wasser und/oder Wasserdampf enthält. Dabei wird im Allgemeinen der Wasserdampf dem Transmissionsmedium zugemischt und/oder der zu sterilisierenden respektive zu desinfizierenden Oberfläche direkt zugeführt. In diesem Zusammenhang hat es sich bewährt, wenn die Temperatur der Oberfläche so eingestellt wird, dass der Wasserdampf bzw. das Wasser an der Oberfläche kondensiert.

Außerdem sollte die Temperatur der Oberfläche gleichmäßig eingestellt werden, um eine homogene Wasserbenetzung der Oberfläche zur Verfügung zu stellen. Tatsächlich hat es sich in diesem Zusammenhang als günstig erwiesen, wenn die Temperatur der Oberfläche so vorgegeben wird, dass Schwankungen von weniger als ± 5°C, insbesondere weniger als ± 2 °C, über die gesamte Oberfläche gesehen, beobachtet werden. Dadurch wird die zu sterilisierende Oberfläche mit Hilfe des Wasserdampfes vollflächig benetzt und kann der Wasserdampf durch die kurzwellige elektromagnetische Strahlung zusätzlich zur Sterilisierung beitragen.

Denn durch die fragliche UV-Strahlung werden im Wasser bzw. dem Wasserdampf OH-Radikale gebildet, die Mikroben abtöten, folglich mikrobiozid wirken. Dabei wird allgemein so vorgegangen, dass das Wasser bzw. der Wasserdampf zu Mikrotröpfchen kondensiert, die beispielsweise Durchmesser im Mikrometerbereich aufweisen. Die fraglichen Wassertröpfchen bilden sich bevorzugt an Keimen, welche in diesem Zusammenhang als Kondensationskeime fungieren. Dadurch werden die gewünschten OH-Radikale exakt örtlich dort gebildet, wo sie benötigt werden, nämlich am Ort der Keime. Es ist also im Allgemeinen nicht erforderlich, dass die gebildeten OH-Radikale zu den Keinem erst noch diffundieren müssen, um dort für deren Abtötung zu sorgen. Vorteilhaft ist weiterhin, dass die durch die Strahlung gebildeten OH-Radikale im Flüssigkeitstropfen einige zehntel bis einige Millieter diffundieren können und damit auch an Orten wirken können, die von der Strahlung nicht direkt bestrahlt werden. Solche Orte können beispielsweise Hinterschneidungen oder Gewindebereiche in einer Kappe sein.

### • Vergleichsbeispiel

Insgesamt hat sich gezeigt, dass im Rahmen der Erfindung die Desinfektionszeit drastisch reduziert werden kann, beispielsweise um den Faktor 5. Geht man nämlich von einer angestrebten Keimreduktion beispielsweise um 5 Dekaden (Verringerung um den Faktor 10⁵) aus, die sich mikrobiologisch ermitteln lässt, so wird im Rahmen der herkömmlichen Vorgehensweise d. h., bei Rückgriff auf eine UV-Strahlungsquelle, eine bestimmte Desinfektionszeit von beispielsweise einer Minute in atmosphärischer Luft benötigt. Dabei mag als UV-Strahlungsquelle beispielsweise eine Niederdruck-Quecksilberdampflampe oder ein Excimerstrahler oder eine andere geeignete Strahlungsquelle, die Quanten bis hinunter zu 170 nm emittiert, eingesetzt werden.

Diese Behandlungszeit von einer Minute lässt sich auf ca. 12 Sek. verringern und der zuvor bereits angesprochene Faktor 5 beobachten, wenn bei ansonsten gleichen Versuchsbedingungen und gleicher angestrebter Keimreduktion um 5 Dekaden die UV-Strahlung von der Strahlungsquelle bis zur Oberfläche nicht atmosphärische Luft passiert, sondern der atmosphärischen Luft als erster Komponente Argon oder ein Argon-Stickstoff-Gemisch als zweite Komponente hinzugefügt werden. Das mag so realisiert werden, dass ein Mischungsverhältnis zwischen der ersten Komponente und der zweiten Komponente im Bereich von ca. 20 Vol.-% zu 80 Vol.-%, bezogen auf das gesamte Volumen des Transmissionsmediums, vorliegt.

Die zuvor erwähnte Zeitverkürzung für die Desinfektion um den Faktor 5 kann noch mal um den Faktor 3 erhöht werden, so dass man unter dem Strich eine Zeitverkürzung um den Faktor 15 beobachtet. D. h. anstelle einer Bestrahlungsdauer von 1 min. werden lediglich noch 4 Sek. bei ansonsten gleichen Bedingungen benötigt. Diese zusätzliche Verringerung der Behandlungszeit bzw. Bestrahlungszeit um den Faktor 3 lässt sich dadurch erreichen, dass dem Transmissionsmedium Wasser respektive Wasserdampf hinzugefügt wird.

Es hat sich als besonders günstig herausgestellt, wenn man dem Transmissionsmedium gerade so viel Wasserdampf hinzugibt, dass man am Packstoff mikroskopisch kleine Kondensationströpchen erhält, deren Durchmesser gerade im Bereich von etwa 0,05 bis 1 mm und vorzugsweise von 0,05 bis 0,3 mm liegt.

Beispielsweise mag bei einem Hohlkörper von 1 l Volumen mit einer zusätzlichen Feuchte in den Flaschen zwischen etwa 100 µg bis 50 mg gearbeitet werden, wobei von der Feuchtemenge nur eine Bruchteil kondensiert und der andere Teil als Dampf im flaschenvolumen vorliegen bleibt. Bevorzugt beträgt die zugegebene Wassermenge gerade soviel, das der kondensierende Bruchteil der Feuchtemenge zwischen 100 µg bis 300 µg. Jedenfalls bewirkt diese zusätzliche Zugabe von Wasser bzw. Wasserdampf im Transmissionsmedium, dass die sterilisierende Wirkung der eingesetzten kurzwelligen elektromagnetischen Strahlung bzw. UV-Strahlung noch einmal vergrößert wird.

Hierbei macht sich die Erfindung die Tatsache zunutze, dass durch die Energie der kurzwelligen elektromagnetischen Strahlung im Wasser OH-Radikale gebildet werden. Diese Effekt ist grundsätzlich bekannt (vgl. den Aufsatz von Moogega Cooper und anderen "Decontamination of Surfaces from Extremophile Organisms Using Nonthermal Atmospheric-Pressure Plasmas"; IEEE Transactions on Plasma Science 2008, S. 1 bis 5). D. h., die geringe Zugabe von Feuchtigkeit zu der ansonsten trockenen Atmosphäre begünstigt die mikrobielle Wirkung der UV-Strahlung an der zu behandelnden Oberfläche.

In diesem Zusammenhang ist die Bildung von OH-Radikalen im Wasser besonders bei kurzen Wellenlängen im Bereich von 200 nm ausgeprägt. Demgegenüber wird die Bildung von OH-Radikalen in Wasser bei größeren Wellenlängen im Bereich von 250 nm reduziert. Da allerdings im Regelfall Argon als zweite Komponente neben Luft in dem Gasgemisch bzw. Argon ausschließlich als Transmissionsmedium eingesetzt wird und dies die Durchlässigkeit bis zu Wellenlängen von ca. 170 nm begünstigt, werden besonders geringe Desinfektionszeiten bei der beschriebenen Kombination (Zusatz von Argon und Wasserdampf) beobachtet.

Im Folgenden wird die Erfindung anhand einer lediglich ein Ausführungsbeispiel darstellenden Zeichnung näher erläutert; es zeigen:
**Fig. 1** eine Vorrichtung zur Sterilisation von Oberflächen, vorliegend in oder an einem Hohlkörper,
**Fig. 2** eine Variante der Fig. 1,
**Fig. 3** die Vorrichtung zur Sterilisation von Oberflächen, und zwar vorliegend von Verpackungsbahnen und
**Fig. 4a bis 4b** die Vorrichtung in einer weiteren Variante zur Sterilisation der Oberflächen einer Kappe als Hohlkörper, wobei hier auf dieDarstellung des in der Kappe befindlichen Gewindes verzichtet wurde.

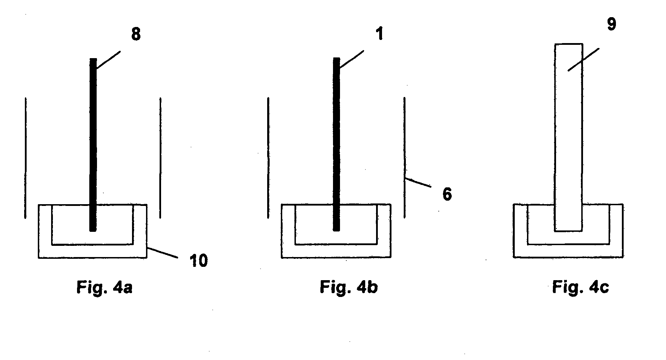

In den Figuren ist eine Vorrichtung zur Sterilisation von Oberflächen dargestellt. Bei den fraglichen Oberflächen handelt es sich im Beispielfall um Verpackungsgutoberflächen, also Oberflächen im Innern oder außen von Verpackungen. Dabei meint im Rahmen der Erfindung Verpackung jede denkbare Umhüllung für beispielsweise Lebensmittel oder auch andere Güter. So können grundsätzlich Hohlkörper 2, 10 auf die nachfolgend noch zu beschreibende Art und Weise an ihren Oberflächen behandelt werden. Bei den Hohlkörpern 2, 10 mag es sich um Dosen, Büchsen, Beutel, Flaschen 2, Becher oder auch Kappen 10 handeln, wie sie in der Fig. 4a dargestellt sind.

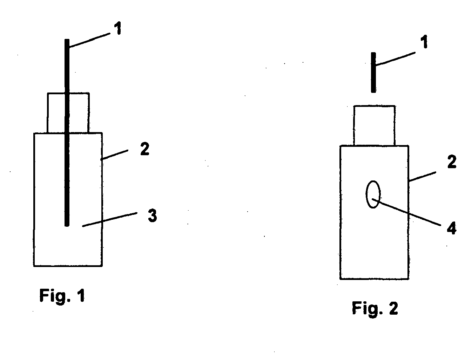

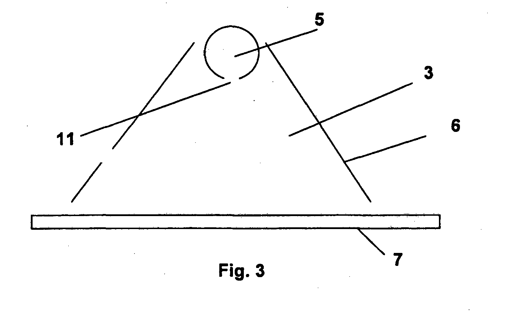

Daneben lassen sich mit Hilfe der nachfolgend noch im Detail zu beschreibenden Vorrichtung auch Oberflächen und insbesondere Verpackungsgutoberflächen von Verpackungsbahnen 7 behandeln bzw. sterilisieren. Eine sterilisierende Behandlung von Oberflächen des Hohlkörpers 2 respektive der Flaschen 2 im Beispielsfall ist in den Fig. 1 und 2 dargestellt. Dagegen beschäftigt sich die Fig. 3 mit der desinfizierenden Behandlung von Oberflächen der Verpackungsbahn 7. Die Fig. 4 ist schließlich gerichtet auf die Behandlung von Oberflächen im Innern oder außen von Kappen 10, die beispielsweise im Anschluss an diese Behandlung zum Verschluss der Flaschen 2 oder von anderen Hohlkörper 2, 10 eingesetzt werden.

Zum grundsätzlichen Aufbau der besagten Vorrichtung gehört zunächst einmal eine kurzwellige elektromagnetische Strahlungsquelle 9, die als UV-Strahlungsquelle bzw. UV-Strahler 9 ausgebildet ist. Im Rahmen der Darstellung nach den Fig. 1, 2 und 3 ist der UV-Strahler 9 nicht ausdrücklich gezeigt. Allerdings wird im Rahmen der Fig. 1, 2 und 3 genauso oder vergleichbar vorgegangen, wie dies die einzelnen Verfahrensschritte nach den Fig. 4a, 4b und 4c grundsätzlich erläutern.

Danach wird die zu behandelnde Oberfläche zunächst in einem ersten Schritt mit Wasser oder Wasserdampf behandelt. Dabei mag das Wasser direkt über ein Tauchrohr 8 für Wasserdampf zugeführt werden. Das Wasser bzw. der Wasserdampf benetzt die zu behandelnde Oberfläche, im Beispielfall nach der Fig. 4a das Innere der dortigen Kappe 10.

Im Anschluss an diesen ersten Verfahrensschritt wird die fragliche Kappe 10 bzw. die zu desinfizierende Oberfläche mit einem Transmissionsmedium beaufschlagt. Grundsätzlich kann das Wasser oder der Wasserdampf dem Transmissionsmedium auch zugemischt werden. Bei diesem Transmissionsmedium handelt es sich im Ausführungsbeispiel um ein Gasgemisch. Das Gasgemisch setzt sich aus atmosphärischer Luft als erster Komponente und Argon als weiterer zweiter Komponente zusammen. Anstelle von Argon kann auch Stickstoff oder ein Argon/Stickstoffgemisch als zweite Komponente Verwendung finden. Jedenfalls bewirkt die zweite Komponente, dass das fragliche Transmissionsmedium insgesamt auf die vom UV-Strahler 9 abgegebene kurzwellige elektromagnetische Strahlung bzw. UV-Strahlung abgestimmt ist. Denn das fragliche Transmissionsmedium verfügt über eine im Vergleich zu an dieser Stelle meistens eingesetzter atmosphärischer Luft geringere Absorption für die UV-Strahlung.

Das Transmissionsmedium kann selbstverständlich auch in reiner Form beispielsweise als Schutzgas für die hierdurch geführte elektromagnetische Strahlung fungieren. Dann sind entsprechende Abschottungsmaßnahmen erforderlich, um eine Mischung mit der umgehenden Luft zu verhindern. Im Allgemeinen findet jedoch - zwangsläufig - eine Luftmischung statt, so dass nachfolgend durchgängig von einem Gasgemisch oder Flüssigkeits-Gasgemisch die Rede ist.

Nachdem das Innere der Kappe 10 im Rahmen der Fig. 4b mit Hilfe des Transmissionsmediums bzw. dem Gasgemisch aus Luft und Argon im Beispielfall gespült worden ist, wird die fragliche und zu behandeinde Oberfläche im Innern der Kappe 10 mit Hilfe der anschließend eingebrachten UV-Strahlungsquelle 9 beaufschlagt. Durch das im Innern der Kappe 10 vorhandene Transmissionsmedium wird die Absorption der von der UV-Strahlungsquelle 9 emittierten UV-Strahlung gegenüber einer vergleichbaren Füllung der Kappe 10 mit atmosphärischer Luft verringert. Dadurch steigt die Strahlungsintensität an der zu behandelnden Oberfläche. Folglich ist auch die UV-Strahlungsdosis erhöht und verbessert sich die keimabtötende Wirkung, wie dies einleitend bereits erläutert wurde.

Da zudem die zu behandelnde Oberfläche im Innern der Kappe 10 mit Wasserdampf benetzt ist bzw. sich an dieser Stelle Mikrotröpfchen aus Wasser gebildet haben, und zwar unter Rückgriff auf dort ohnehin vorhandene Keime als Kondensationskeime, wird die zuvor im Detail erläuterte Bildung von OH-Radikalen im Wasser durch die Einwirkung der UV-Strahlung begünstigt. Dadurch wird die keimabtötende Wirkung der UV-Strahlung nochmals erhöht und es kommt zu der einleitend bereits beschriebenen deutlichen Verringerung der Desinfektionszeit, die im Vergleich zu einer trocknenden Luftatmosphäre um bis zu den Faktor 10 oder noch mehr verringert ist.

In diesem Zusammenhang empfiehlt es sich, die zu behandelnde Oberfläche auf eine bestimmte Temperatur einzustellen, um eine homogene Benetzung der Oberfläche mit dem eingeleiteten Wasserdampf zu erzielen. Dabei wird man die Temperatur der Oberfläche gleichmäßig über die gesamte Fläche so einstellen, dass beispielsweise Temperaturabweichungen im Bereich von allenfalls ± 2°C über die gesamte Oberfläche gesehen beobachtet werden.

Die Einstellung der Oberflächentemperatur der zu desinfizierenden Oberfläche mittels einer Oberflächenheizung ist auch vor dem Hintergrund günstig, dass ausweislich der Fig. 2 nicht nur eine Wasserbenetzung möglich ist und durchgeführt wird, wie sie in der Fig. 4a mit dem dortigen Tauchrohr 8 für Wasserdampf gezeigt ist. Sondern das Transmissionsmedium kann auch als Flüssigkeits-/Gasgemisch ausgelegt sein. Dann wird unverändert atmosphärische Luft als erste Komponente eingesetzt bzw. liegt zwangsläufig vor. Allerdings handelt es sich bei der weiteren zweiten Komponente um eine Flüssigkeit, die nach ihrem Verdampfen ein Schutzgas bildet, welches eine im Vergleich zu atmosphärischer Luft verringerte Absorption der kurzwelligen elektromagnetischen Strahlung zeigt.

Beispielsweise mag es sich hier um eine zweite flüssige Komponente 4 handeln, die ins Innere des Hohlkörpers 2 eingebracht wird (vgl. Fig. 2). Denkbar ist es in diesem Zusammenhang beispielsweise, in den Hohlkörper 2 wenige Milliliter flüssiges Argon oder flüssigen Stickstoff über ein Tauchrohr 1 (oder ein anderes Tauchrohr) zu injizieren. Die fragliche zweite flüssige Komponente 4 bzw. das in flüssiger Form eingebrachte Schutzgas 4 verdampft in kurzer Zeit (weniger als 5 Sek.) vollständig und füllt den Hohlkörper bzw. die Flasche 2 überwiegend aus. Dabei wird die beschriebene Verdampfung noch für den Fall begünstigt, dass der Hohlkörper bzw. die Flasche 2 eine bestimmte (erhöhte) Oberflächentemperatur von beispielsweise 40°C oder auch 50°C aufweist. - Alternativ hierzu zeigt die Fig. 1, wie über das Tauchrohr 1 die gasförmige zweite Komponente bzw. das Schutzgas in gasförmiger Form in die Flasche 2 eingebracht wird und die dort vorhandene Luft nahezu vollständig verdrängt.

Jedenfalls mag im Anschluss an die Beaufschlagung des Hohlkörpers 2 mit dem Transmissionsmedium bzw. Gasgemisch nach Fig. 1 oder auch nach Verdampfen der zweiten flüssigen Komponente 4 oder des flüssigen Schutzgases 4 (flüssiges Argon und/oder flüssiger Stickstoff) entsprechend dem Beispiel nach Fig. 2 im Anschluss hieran die UV-Strahlungsquelle 9 in den Hohlkörper 2 zwecks Desinfektion ähnlich eingeführt werden, wie dies in der Fig. 4c grundsätzlich dargestellt ist.

Im Rahmen der Fig. 3 und 4 erkennt man dann noch eine Leiteinrichtung 6, welche grundsätzlich dazu dient, das beispielsweise aus dem Tauchrohr 1 oder aus dem Längsrohr 5 über dortige Austrittsöffnungen 11 austretende Transmissionsmedium bzw. das Gasgemisch im Beispielfall zu führen. Dabei wird man im Rahmen der Variante nach Fig. 3 so vorgehen, dass die UV-Strahlungsquelle 9 durch den von der Leiteinrichtung 6 bzw. den beiden beidseitig des Längsrohres 5 angeordneten Leitblechen 6 gebildeten Vorhang hineinstrahlt. In diesem Zusammenhang kann erneut Wasser oder Wasserdampf auf die dortige Verpackungsbahn 7 aufgebracht werden. Zu diesem Zweck mag die Leiteinrichtung 6 auf eine Temperatur gebracht werden, die beispielsweise um 5°C höher oder noch höher angesiedelt ist als die Temperatur der Verpackungsbahn 7, um auf jeden Fall eine Kondensation des Wassers bzw. des Wasserdampfes auf der Oberfläche der Verpackungsbahn 7 zu realisieren.

Die Verpackungsbahn 7 ist als bahnförmiges Verpackungsmaterial ausgeführt, beispielsweise als Kunststoffbahn, Folienbahn, Bahn aus Verbundmaterialien etc.. Mit Hilfe derartiger Verpackungsbahnen 7 lassen sich beispielsweise einzelne Gebinde umschlingen und können die die Gebinde bildenden Hohlkörper zusammengefasst werden. Dabei ist es oftmals erforderlich, die fragliche Verpackungsbahn 7 wie beschrieben zu sterilisieren.

Das Wasser bzw. der Wasserdampf kann grundsätzlich dem Transmissionsmedium hinzugefügt werden. Dann wird man beispielsweise so vorgehen wie in der Fig. 1 oder auch in der Fig. 2 oder 3 gezeigt. Es ist aber auch möglich, die Beaufschlagung der zu desinfizierenden Oberfläche in mehreren. Schritten vorzunehmen. Dann wird man zunächst die Oberfläche mit Wasser oder Wasserdampf beaufschlagen, wie dies in der Fig. 4a dargestellt ist. Darauffolgend wird dann die Oberfläche mit dem Transmissionsmedium gespült bzw. der an dieser Stelle realisierte Hohlkörper 2 respektive die Kappe 10 oder Flasche 2 mit dem Transmissionsmedium in ihrem Innern gefüllt. Danach erfolgt erst die Behandlung mit der UV-Strahlungsquelle 9. Wie bereits erläutert, können die Schritte der Wasserdampf- und Transmissionsmediumzuführung selbstverständlich auch umgekehrt werden. Außerdem lassen sich beide Schritte auch kombinieren.

## Patentansprüche

1. Verfahren zur Sterilisation von Oberflächen, insbesondere von Verpackungsgutoberflächen, beispielsweise Oberflächen von Verpackungsbahnen (7), Hohlkörpern (2, 10) oder dergleichen, wonach
- kurzwellige elektromagnetische Strahlung die Oberfläche beaufschlagt, wobei
- die Strahlung von einer Strahlungsquelle (9) bis zur Oberfläche durch ein auf die Strahlung abgestimmtes Transmissionsmedium geführt wird, indem
- als Transmissionsmedium mindestens ein Gas und/oder beispielsweise ein atmosphärische Luft enthaltendes Gasgemisch eingesetzt wird, wobei
- das als reines Gas oder zweite Komponente eingesetzte Gas eines Gasgemisches wenigstens eine gegenüber Luft geringere Absorption der kurzwelligen elektromagnetischen Strahlung aufweist, und wobei
- als weiteres Transmissionsmedium eine Flüssigkeit eingesetzt wird,
wobei Wasser und/oder Wasserdampf dem Transmissionsmedium und/oder der zu desinfizierenden Oberfläche zugeführt wird, **dadurch gekennzeichnet, dass** das reine Gas oder
die zweite Komponente ein Edelgas ist, welches in gasförmiger und/oder flüssiger Form der Luft und/oder der zu desinfizierenden Oberfläche zugeführt wird.

2. Verfahren nach Anspruch 1, **dadurch gekennzeichnet, dass** die zu desinfizierende Oberfläche beheizt wird, um gegebenenfalls die Verdampfungsgeschwindigkeit der zweiten Komponente bzw. des flüssigen Edelgases zu erhöhen.

3. Verfahren nach Anspruch 1 oder 2, **dadurch gekennzeichnet, dass** die Temperatur der Oberfläche so eingestellt wird, dass das Wasser und/oder der Wasserdampf an der Oberfläche kondensiert.

4. Verfahren nach einem der Ansprüche 1 bis 3, **dadurch gekennzeichnet, dass** die Temperatur der Oberfläche gleichmäßig eingestellt wird, um eine homogene Wasserbenetzung der Oberfläche zu erzielen.

5. Verfahren nach einem der Ansprüche 1 bis 4, **dadurch gekennzeichnet, dass** die Temperatur der Oberfläche so eingestellt wird, dass Schwankungen von weniger als +- 5 °C, insbesondere weniger als +- 2 °C, über die Oberfläche gesehen beobachtet werden.

## Claims

1. Method for sterilising surfaces, especially surfaces of packaging material, e.g. surfaces of packaging webs (7), hollow elements (2, 10), or the like, according to which
- short-wave electromagnetic radiation impinges on the surface, wherein
- the radiation is guided from the source of radiation (9) to the surface by a transmission medium that is adjusted to the radiation, by
- using, as a transmission medium, at least a gas and/or e.g. a gas mixture containing atmospheric air, wherein
- the gas, used as a pure gas or second component, of a gas mixture has at least a lower absorption, with respect to air, of the short-wave electromagnetic radiation, and wherein
- a liquid is used as a further transmission medium
wherein water and/or water vapour is supplied to the transmission medium and/or the surface to be disinfected, **characterised in that** the pure gas or the second component is a noble gas which, in gaseous and/or liquid form, is supplied to the air and/or the surface to be disinfected.

2. Method according to claim 1, **characterised in that** the surface to be disinfected is heated in order to increase, where required, the speed of evaporation of the second component or of the liquid noble gas.

3. Method according to claim 1 or 2, **characterised in that** the temperature of the surface is adjusted such that the water and/or the water vapour condenses on the surface.

4. Method according to any one of claims 1 to 3, **characterised in that** the temperature of the surface is adjusted evenly in order to achieve homogeneous water wetting of the surface.

5. Method according to any one of claims 1 to 4, **characterised in that** the temperature of the surface is adjusted such that variations of less than +/- 5°C, in particular less than +/- 2°C, are observed seen across the surface.

## Revendications

1. Procédé de stérilisation de surfaces, en particulier de surfaces de produit d'emballage, par exemple de surfaces de bandes d'emballage (7), de corps creux (2, 10) ou similaires, selon lequel
- la surface est soumise à l'action d'un rayonnement électromagnétique à ondes courtes, sachant que
- le rayonnement est guidé par une source de rayonnement (9) jusqu'à la surface par un milieu de transmission adapté au rayonnement, en ce
- qu'on utilise comme milieu de transmission au moins un gaz et/ou par exemple un mélange gazeux contenant un air atmosphérique, sachant
- que le gaz utilisé comme gaz pur ou deuxième composant d'un mélange gazeux présente au moins une absorption du rayonnement électromagnétique à ondes courtes plus faible par rapport à l'air, et sachant
- qu'on utilise comme milieu de transmission un liquide,
- sachant que l'eau et/ou la vapeur d'eau sont amenées au milieu de transmission et/ou à la surface devant être désinfectée,
**caractérisé en ce que** le gaz pur ou le deuxième composant est un gaz noble, qui est amené sous forme gazeuse et/ou sous forme liquide à l'air et/ou à la surface devant être désinfectée.

2. Procédé selon la revendication 1, **caractérisé en ce que** la surface devant être désinfectée est chauffée afin d'augmenter éventuellement la vitesse d'évaporation du deuxième composant ou du gaz noble liquide.

3. Procédé selon la revendication 1 ou 2, **caractérisé en ce que** la température de la surface est réglée de telle manière que l'eau et/ou la vapeur d'eau se condensent à la surface.

4. Procédé selon l'une quelconque des revendications 1 à 3, **caractérisé en ce que** la température de la surface est réglée de manière homogène afin d'obtenir une humification de la surface à l'eau homogène.

5. Procédé selon l'une quelconque des revendications 1 à 4, **caractérisé en ce que** la température de la surface est réglée de telle manière qu'on observe des fluctuations au-dessus la surface inférieures à +-5 °C, en particulier inférieures à +-2 °C.
